# EUROPEAN PATENT APPLICATION

(11) **EP 0 815 818 A1**
(43) Date of publication of application: **07.01.1998**
(21) Application number: 96115445.7
(22) Date of filing: 26.09.1996
(51) Int. Cl.: A61F 13/15

(54) **Concentrating liquid absorption to the center of absorbent articles**

(30) Priority: 01.07.1996 EP 96110575
(71) Applicant: THE PROCTER & GAMBLE COMPANY, Cincinnati, Ohio 45202 (US)
(72) Inventor: Carlucci, Giovanni, 66100 Chieti (IT); Giorgini, Gennaro, 64026 Roseto (IT); D'Alessio, Nicola, 65126 Pescara (IT)
(74) Representative: Hirsch, Uwe Thomas

(57) **Abstract**

The present invention relates to the use of an absorbent expanding material in an absorbent article, such as a sanitary napkin in order to concentrate the absorbed liquid in the central region of the absorbent article. In particular the present invention relates to the use of an absorbent expanding material which upon absorption expands more than the absorbed volume of liquid requires in order to provide additional void volume. In addition the present invention relates to absorbent articles such as baby diapers, adult incontinence products or sanitary articles in which the material of the absorbent core has a better liquid transport capability into the thickness of the article than within the plane of the article.

## Description

### Field of the invention

The present invention relates to the use of an absorbent expanding material in an absorbent article, such as a sanitary napkin in order to concentrate the absorbed liquid in the central region of the absorbent article. In particular the present invention relates to the use of an absorbent expanding material which upon absorption expands more than the absorbed volume of liquid requires in order to provide additional void volume. In addition the present invention relates to absorbent articles such as baby diapers, adult incontinence products or sanitary articles in which the material of the absorbent core has a better liquid transport capability into the thickness of the article than within the plane of the article.

### Background of the invention

Disposable absorbent articles such as sanitary napkins or baby diapers or adult incontinence articles or pantiliners are well-known. Usually these articles comprise a wearer facing surface provided by a topsheet, a garment facing surface provided by a backsheet and an absorbent core between the topsheet and the backsheet for absorption of liquids. The topsheet naturally is liquid permeable in order to allow absorption of the liquid by the absorbent core. The backsheet is designed to prevent loss of absorbed liquids on the garment facing side of the absorbent article and therefore can be totally impermeable to liquids and gases or maybe provided in such a way that liquid loss is prevented while breathability or at least vapour permeability is provided.

It has now been found that the use of expanding materials in the absorbent core of absorbent articles is beneficial since the initial product can be provided in a thin typically planar format while the product expands during use in order to create the volume in which the liquids to be absorbed are stored. Typical examples for such expanding materials are so called absorbent gel materials also called super absorbents or compressed materials which are released upon being wetted from compressed state. Compressed fibrous structures or sponge structures, in particular regenerated cellulose sponge materials have been described for example in US 3,736,931, US 3,512,530, EP-293 208, European application EP-96106724.6 and EP-96106723.8.

Absorbent gel materials are typically provided in a distributed particulate form such that the absorbent core is substantially larger than the area in which the liquid uptake takes place. The particulate absorbent gel material is also usually provided together with cellulose fibers or tissues to ensure good liquid transport to the particles and provide additional absorption capacity.

It is common in the art to include in the absorbent core means for distributing the liquid to be absorbed in order to better utilise the total absorbent structure. This is of course useful provided such liquid distribution layers do not transfer the liquid to the periphery of the article where side leakage may occur. Means for preventing such side leakage have been developed in particular in the diaper field and have been commercialised on diapers as waist shields or waist bands.

Also in sanitary napkins it was suggested that side leakage should be reduced and hence special designs to reduce the leakage out of the peripheral edges of the sanitary napkin or pantiliner product have been developed. For example EP-B-304 957 which discloses a sanitary napkin having barrier seals across the absorbent core in transverse direction in order to prevent the absorbed liquid from spreading along the length of the sanitary napkin such that no leakage at the front and rear end can occur.

In this product side flaps are also provided which are folded around the edges of the undergarment in which such an article is worn. The side flaps are also separated from the central portion of the absorbent structure by use of a barrier seal in order to prevent liquid wicking from the central portion of the absorbent article into the side flaps. Also EP-A-301 491 discloses fluid barrier seals which are designed to prevent liquid migration from a central portion of the absorbent article to a more peripheral portion in this case the side flaps of the sanitary napkin.

It is hence an established problem that common designs of absorbent articles, notable sanitary napkins, having an absorbent structure with good liquid distribution capability can suffer from potential side edge and/or end edge leakage.

It has only now been found that absorbent expanding materials in the absorbent structure of disposable absorbent articles can provide an unexpected benefit in respect to maintaining the periphery of the disposable absorbent article less likely to acquire liquid by internal liquid transport and thereby to reduce probability of liquid leaking from the absorbent article.

### Summary of the invention

The present invention relates to the use of an absorbent expanding material in an essentially planar absorbent article. The essentially planar absorbent article comprises a topsheet, a backsheet and an essentially planar absorbent core disposed between the topsheet and the backsheet. The absorbent core comprises the absorbent expanding material.

Within the plane of the absorbent core a central region can be defined which according to the present invention is the region into which liquid is initially absorbed through the topsheet under usual usage conditions of such an article. From that central region a peripheral region can be distinguished which is outside the central region within the plane of the absorbent core of the absorbent article. According to the present invention the absorbent expanding material is used to concentrate the absorbed liquid in the central region of the absorbent core. In particular it is preferred that the absorbent expanding material expands upon initial absorption of liquid by a volume which is larger than the volume of the absorbed liquid itself and thereby creates additional void volume in the central region of the absorbed core.

Preferably the absorbent expanding material extends throughout the central region of the absorbent core in order to allow for the variation in habits of the user of such articles. Even more preferred is the use of absorbent cores which comprise the absorbent expanding material only in the central region such that it does not extend into the peripheral region. Thereby the absorbent core and the absorbent expanding material are not coextensive within the plane of the planar absorbent core.

In principle it does not matter in which direction the absorbent expanding material expands when it absorbs liquid. However, it is preferred to use the material such that it expands in a direction from the absorbent core towards the topsheet in order to form the article into a tridimensional structure thereby raising the topsheet towards the wearer of such articles and increasing the probability of early interception of liquid disposed on the article after the initial absorption of liquid.

The absorbent expanding material preferably comprises a regenerated cellulose sponge material and preferably even consists of a regenerated cellulose sponge material. It is most preferred to use the regenerated cellulose sponge material according to the present invention if the regenerated cellulose sponge material comprises incisions which maintain the absorbent expanding material still intact as a unitary structure but provide for increased flexibility of the sponge material while also decoupling incremental fractions of the sponge material from neighbouring incremental fractions of the sponge material in order to liberate the full expanding capability of the sponge material. In an even more preferred embodiment of the present invention the regenerated cellulose sponge material in addition to the incisions also has apertures preferably on the surface of the regenerated cellulose sponge material which is closest to the topsheet.

The use of the absorbent expanding material according to the present invention to concentrate the absorbed liquid in the central region of the absorbent core is most suitably achieved when reducing or even eliminating all possible distribution materials. In a preferred embodiment an article in which at least 50 %, preferably at least 80 %, most preferably ate least 95 %, by volume of the material of the absorbent core, which extend beyond the central region of the absorbent core, have a maximum planar liquid transport capability in a direction within the essentially planar absorbent core which is not larger than their maximum perpendicular liquid transport capability in a direction perpendicular to the essentially planar absorbent core. In other terms the absorbent core as a whole should be better at transporting liquid into the absorbent core than spreading it. This can best be achieved by not including so called wicking or distribution layers or limit such layers to the central region of the absorbent core.

In a product aspect according to the present invention an absorbent article for absorbing liquids and comprising a topsheet, a backsheet and an absorbent core according to the above definitions comprises an absorbent expanding material to maintain the absorbed liquid concentrated in the central region of the absorbent core which article is characterised in that each material comprised in the absorbent core has a maximum planar liquid transport capability in a direction within the essentially planar absorbent core and each material also has a maximum perpendicular liquid transport capability in a direction perpendicular to the essentially planar absorbent core. This article is further characterised in that the value of the maximum planar liquid transport capability is smaller than the value of the maximum perpendicular liquid transport capability for at least 50 %, preferably at least 80 %, most preferably at least 95 %, by volume of the material which extends into the peripheral region. Preferably such articles are baby diapers or adult incontinence articles.

### Brief description of the drawings

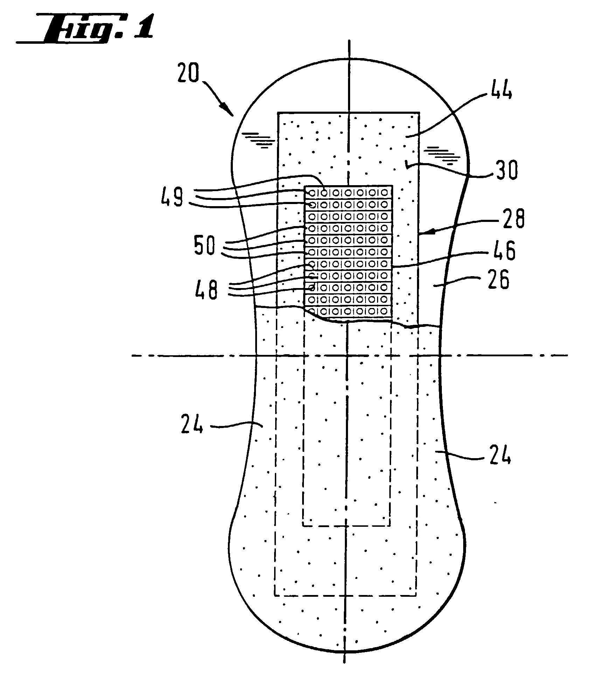

Figure 1 shows a sanitary napkin which is preferred for the use according to the present invention.

### Detailed description of the invention

This invention relates to a disposable absorbent article which article exhibits absorbency for bodily fluids, the protection of the user's garments from soiling, improved physical comfort to the user, and which is easy to produce and to package. The disposable absorbent article is described below by reference to a sanitary napkin or catamenial, however baby diapers or adult incontinence articles are also included under the term disposable absorbent articles. The term "sanitary napkin", as used herein, refers to an article which is worn by females adjacent to the pudendal region and which is intended to absorb and contain the various body fluids which are discharged from the body (e.g., vaginal discharges, menses, and/or urine) and which is intended to be discarded after a single use. The disposable absorbent article is substantially flat or essentially planar prior to use.

The term "substantially flat" or "essentially planar", as used herein, refers to articles that have their main extension in one plane in contrast to being shaped. In a preferred embodiment an essentially planar article will have an absorbent core of constant thickness or, at least, will have an absorbent core that is essentially not shaped in a direction which is orthogonal to the absorbent core itself. This does not exclude a general curvature of the absorbent core. It will be apparent to the man skilled in the art to which extent products can deviate from an absolute flat shape and still benefit from the present invention.

Sanitary napkins with longitudinal side cuffs, which may be optionally elasticated, and sanitary napkins with a moderate curvature are therefore within the scope of the present invention, provided that their absorbent core is essentially not shaped prior to use in a direction that is orthogonal to the absorbent core itself. However, shaped absorbent cores to better conform to the width of the pudendal region, i.e. having a reduced width versus the front and/or rear end of the core, are generally desirable.

The terms "joined" or "affixed", as used herein, encompasses configurations whereby a first member is directly connected to a second member and configurations whereby a first member is indirectly connected to a second member by connecting the first member to intermediate members which in turn are connected to the second member.

In FIG. 1 a preferred embodiment of a sanitary napkin 20 of the present invention is shown. Fig. 1 is a plan view of the sanitary napkin 20 of the present invention in its flat-out state prior to use with portions of the structure being cut-away to more clearly show the construction of the sanitary napkin 20 and with the portion of the sanitary napkin 20 which faces or contacts the wearer oriented towards the viewer. As shown the sanitary napkin 20 preferably comprises a liquid pervious topsheet 24, a liquid impervious backsheet 26 joined to the topsheet 24, and an absorbent core 28 intermediate the topsheet 24 and the backsheet 26; the absorbent core 28 comprises an expanding layer 46 for expanding the sanitary napkin into a tridimensional structure while being worn by a user. The sanitary napkin has two main surfaces, a body contacting or facing surface, and a garment facing or contacting surface.

Tridimensional structures of the sanitary napkin 20 are those in which the sanitary napkin structure is caused to expand, at least partially in order to more closely conform to the user's anatomy. Particularly preferred are tridimensional structures with a convex upward configuration that are inclusive of, but not limited to, inverted U-shapes or inverted V-shapes, with "convex upward configuration" being meant a structure of the sanitary napkin that is convex on its body facing surface. With these configurations the cross-sectional contour of the central portion of the sanitary napkin more closely matches the labia of the typical wearer.

The topsheet 24 is compliant, soft feeling, and non-irritating to the wearer's skin. Further, the topsheet 24 is liquid pervious, permitting liquid to readily penetrate through its thickness. A suitable topsheet 24 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibres (e.g., wood or cotton fibers), synthetic fibres (e.g., polymeric fibres such as polyester, polypropylene, or polyethylene fibres); or from a combination of natural and synthetic fibres.

A preferred topsheet for the absorbent article of the present invention is a formed film described in one or more of the above patents and marketed on sanitary napkins by The Procter & Gamble Company of Cincinnati, Ohio as "DRI-WEAVE". In a preferred embodiment of the present invention, the body or exposed surface of the formed film topsheet is hydrophilic so as to help liquid transfer through the topsheet faster than if the body surface were not hydrophilic so as to diminish the likelihood that fluid will flow off the topsheet rather than flowing into and being absorbed by the absorbent core.

The topsheet (24) of the present invention is preferably capable of expanding as the sanitary napkin 20 expands in a tridimensional structure upon absorption of body fluids. This may be achieved when the topsheet is made of a material that is intrinsically extensible or by providing it with pleats or folds which may be generally longitudinally, or laterally oriented, or in a combination of these two directions.

The backsheet 26 is substantially impervious to liquids (e.g., menses and/or urine) but otherwise similar to the topsheet. The function of the backsheet 26 is to prevent exudates which may be expelled from or which inadvertently bypass the absorbent core 28 from contacting and soiling the user's undergarments. The backsheet 26 can thus comprise a woven or nonwoven material, polymeric films such as thermoplastic films of polyethylene or polypropylene, or composite materials such as a film-coated nonwoven material. Preferably, the backsheet is a polyethylene film which is embossed and/or has a matte finished to provide a more clothlike appearance. Preferably, the backsheet also provides breathability to the absorbent article by being at least water vapour permeable, preferably air permeable, however, without compromising the main function of the backsheet. The backsheet can be a laminate material e.g. of a microporous film and apertured formed film. Breathability if desired can be limited to the periphery of the backsheet or it can be across the whole backsheet.

In a preferred embodiment of the present invention the sanitary napkin 20 is also provided with a panty fastening means. Typically, at least a portion of the outer surface of the backsheet 26 is coated with adhesive to form a panty fastening adhesive.

The panty fastening adhesive is typically covered with a removable release paper or film in order to prevent the adhesive from drying out or adhering to another surface other than the panty prior to use.

In a preferred embodiment the absorbent core 28 comprises the expanding layer 46 and a separate, substantially non expanding absorbent element 44 joined together and in face to face relationship to each other, the expanding layer 46 being positioned between the topsheet 24 and the absorbent element 44.

The absorbent element 44 and the expanding layer 46 may be associated in any suitable manner to form the absorbent core 28. Suitable manners include, but are not limited to, associating the absorbent element 44 and the expanding layer 46 with adhesives such as by spray-gluing or by applying lines or spots of adhesive between them. Alternatively, or additionally, the association between the layers may be achieved by fibre entanglement or by a plurality of discrete fusion bonds.

Alternatively, the expanding layer 46 may constitute the entire absorbent core 28.

The absorbent capacity of the absorbent core 28 should be compatible with the intended body fluid loading for the absorbent article including the volume created by the expansion of expanding layer.

If present, as illustrated in FIG. 1 the substantially non expanding absorbent element 44 of the absorbent core 28 can be any absorbent means which is generally compressible, resilient, non-irritating to the wearer's skin and capable of absorbing and containing body fluids. The absorbent element 44 may be manufactured from a wide variety of liquid absorbent materials commonly used in disposable absorbent articles.

In the embodiment illustrated in FIG. 1 the absorbent element 44 of the absorbent core 28 comprises an absorbent laminate layer made of a thermally bonded airlaid material longitudinally folded twice on itself and comprising particles of absorbent gelling material therebetween.

The absorbent core 28 comprises an expanding layer 46 for expanding the sanitary napkin 20 while the sanitary napkin 20 is being worn to concentrate the absorbed liquid in the central region of the article. In a preferred embodiment the expansion is provided by swelling of expanding layer 46 and is activated during wear by the initial absorption of body fluids. The expanding layer 46 may comprise any material that is capable of such swelling, such as fibrous material or structures of cells which have been compressed or otherwise put into a state of reduced volume relative to their volume after initial exposure to liquid. This includes but is not limited in particular to fibrous substrates comprising super absorbent fibers, fibers coated with absorbent gelling material suspended in a matrix of compressed resilient fibers, compressed foams or sponges from natural or artificial sources, or compressed peat mass.

Other swelling materials, such as particulate absorbent gel materials or super absorbents, non collapsible but form stable fibrous structures, e.g. bonded polymeric networks or scrims can also be used according to the present invention but are less preferred.

After the absorption of body fluids and the subsequent swelling, the material of the expanding layer 46 must remain soft, compliant, conformable and resilient. The ability to follow the topography of the anatomy will provide intimate contact with the exposed genitalia of the female user. This helps provide better fluid transfer from the user into the expanding layer 46. While these characteristics of the expanding layer 46 allow for improved fit, they also cause the product to concentrate the absorbed liquid in the center of the article.

It is preferred that the expanding layer 46 forms at least part of that surface of the absorbent core 28 facing the topsheet. In the embodiment illustrated in FIG. 1 the expanding layer 46 is positioned over the absorbent element 44, in face to face relationship.

The expanding layer 46 of the absorbent core 28 has a body facing surface oriented towards the topsheet and a garment facing surface opposed thereto, and preferably comprises incisions 48 on at least one of its body facing surface or its garment facing surface that can be arranged in a closed array or in an open array of intersecting lines.

The term "incision", as used herein, indicates a cut in the surface of a layer; incisions can involve the whole thickness of the layer, but it is intended that they cannot divide the layer into two or more separate portions.

A closed array of intersecting lines is an array in which the lines are arranged in a network and therefore are completely interconnected to one another, as illustrated in FIG. 1. In an open array of intersecting lines the lines can intersect to form closed contours, e.g. square, circular, hexagonal contours, but do not form an interconnected network.

The incisions can have a depth which preferably ranges from 50% to 95% of the thickness of the expanding layer 46 in its dry, not-expanded state, more preferably from 70% to 90%; the pitch of the array is between 0.5 and 10 mm.

As shown in FIG. 1 the incisions 48 are preferably arranged in a closed array of intersecting lines in order to divide the body facing surface of the expanding layer 46 into a number of smaller elements 50 connected to each other at their bottom, that is, at the garment facing surface of the expanding layer 46. Upon activation by absorbed fluid each element 50 can swell without being restrained by the surrounding zones of the expanding layer 46 that have not yet been reached by the fluid. This allows a much longer swelling of localised zones of the expanding layer 46 upon absorption when the first drops of fluid are received by the expanding layer 46. This then supports the formation of additional void volume in the center to provide the concentration of absorbed liquid there.

The expanding layer 46 of the absorbent core 28 further comprises apertures 49 in at least one of its body facing surface or garment facing surface. The apertures extend more than 30% of the thickness of the expanding layer 46, preferably more than 50%, more preferably more than 80%; apertures having different depths distributed in various zones of the expanding layer 46 are also possible. The apertures 49 can also extend through the whole thickness of the expanding layer 46.

The total area of the apertures 49 constitutes from 5% to 80%, preferably from 10% to 65%, more preferably from 15% to 45% of the surface area of the body facing surface or of the garment facing surface of the expanding layer 46 in which they are comprised; this corresponds to the open area of said surfaces.

The apertures 49 may be disposed in the expanding layer 46 at random or arranged in a repetitive pattern; preferred patterns for the apertures 49 may be squared array patterns, as illustrated in FIG. 1. When, as it is preferred, apertures and incisions are both present on a same surface of the expanding layer 46, they can be arranged in any way, e.g., the apertures can intersect the incisions; preferably, as illustrated in FIG. 1 each smaller element 50 comprises an aperture 49.

The combined effect of incisions 48 and apertures 49 both provided on the body facing surface of the expanding layer 46 is that the first release of fluid is rapidly acquired within the apertures 49 which provide the expanding layer 46 with a larger void space for initial fluid acquisition and with a larger surface area available for the fluid absorption.

Upon absorption of this first amount of body fluid, the smaller elements 50 of the expanding layer 46 are capable of swelling substantially independently from the surrounding elements not yet reached by the fluid, without being restrained by them. The zone of the expanding layer 46 that first receives the fluid may therefore swell more than if it were not divided in elements 50 separated from one another by the incisions 48.

In a preferred embodiment of the present invention the expanding layer 46 comprises a sheet of compressed regenerated cellulose sponge.

The regenerated cellulose sponge that preferably constitutes the expanding layer 46 is a material that is known in the art; examples of suitable materials are described in U.S. Patent No. 3,954,493, in French Patent Application FR-A-2,203,827, and in European Patent EP-B-0 293 208. The regenerated cellulose sponge is a sponge of a material containing a cellulose skeleton. Examples of such sponges include, in addition to sponges consisting of cellulose itself, sponges consisting of a cellulose derivative as viscose, a cellulose ether and a cellulose ester, and sponges consisting of mixtures of those materials.

By way of example only, a regenerated cellulose sponge may be prepared from a mixture of a viscose solution with reinforcing fibres and a porogenic compound, e.g. crystals of sodium sulphate decahydrate or of another alkali metal salt with a high content of crystallized water, the final pore dimension being related to that of the salt crystals. The viscose solution may be extruded through an extrusion die of the desired section, then let coagulate. The material is washed with water after regeneration in order to eliminate the salt and other possible soluble compounds, then it is dried and, if necessary, compressed to the desired density.

The compressed regenerated cellulose sponge has a network structure that contains air bubbles created by the elimination of the sodium sulphate crystals.

The compressed regenerated cellulose sponge material is available in various forms, e.g. in layers or sheets of different densities, thicknesses and basis weights; dry densities values for the compressed material used in the present invention are from 0.1 g/cc to 1 g/cc, while thicknesses can range from 0.2 mm to 5 mm.

The swelling upon liquid absorption of the compressed regenerated cellulose sponge material that forms the expanding layer 46 takes place substantially in the direction of the compression and restores the pore size of the regenerated sponge before the compression; it creates a void volume that does not collapse in wet conditions and therefore enables the material to rapidly acquire further releases of fluid.

In the embodiment illustrated in FIG. 1 the sanitary napkin 20 comprises an expanding layer 46 made of a sheet of compressed regenerated cellulose sponge, and an absorbent element 44.

The compressed regenerated cellulose sponge has a dry density of 0.5 g/cc and a thickness of 2 mm. The absorbent element 44 of the absorbent core 28 is 207 mm long and 64 mm wide, and the expanding layer 46 of compressed regenerated cellulose sponge is 125 mm long and 30 mm wide. The incisions 48 are arranged on the body facing surface of the expanding layer 46 in a squared array having a 4 mm pitch defining squared smaller elements 50. The depth of the incisions is about 1.7 mm. The expanding layer 46 further comprises apertures 49 made through the whole thickness of the layer 46 and having a circular shape with a diameter of 2 mm that is constant through the thickness of the layer. The apertures 49 are arranged in such a way that each smaller element 50 comprises a single aperture 49, with a resulting open area of 20%. Suitable sheets of compressed regenerated cellulose sponge may be those produced by Spontex France.

The compressed regenerated cellulose sponge sheet 46 is capable of absorbing body fluids quickly with an increase in its volume from about 2 to 20 times, and usually from 5 to 15 times its volume at the time of the compression. The volume increase substantially corresponds to a swelling in the direction of the compression.

The concentration of absorbed liquid in the center region of the article can be further optimised in dependence on the capability of liquid transport of the materials used in the absorbent core construction. The "capability of liquid transport" according to the present application is the speed of mass transport in a direction of a material. This can be quantified by measuring the quantity and speed of wicking within a material. It is important to consider both the speed of transport and the quantity transported such that a value for the liquid transport would indicate quantity of liquid transported per time increment through a defined cross-section of the material. This can be measured for example against gravitational forces or for horizontal wicking.

The absolute capability of liquid transport is only in so far relevant as it should provide the article with the desired speed of absorbency. This will easily be assessed by those skilled in the art when comparing to common materials for absorption or trial of the article.

However, according to the present invention the relative capability of liquid transport through the thickness of the material versus the liquid transport capability within the plane of a material is of concern. In order to optimise the concentration of liquids in the center of the article it is desirable to have a higher or equal liquid transport capability through the article than along the plane of the article.

If this criterium is met by at least 50 %, preferably at least 80 %, most preferably at least 95 %, by volume of the material used in the construction of the absorbent core concentration of the absorbed liquid in the center of the article is improved. Particularly good results can be achieved when the combined liquid transport capability of all materials in the absorbent core through the article is 5 %, preferably 10 % more than the maximum liquid transport capability in any direction within the plane of the absorbent article.

Liquid transport capability can be measured with water or by use of an isotonic sodium chloride solution. Use of liquids similar to those intended for absorption is also possible but usually not required in order to provide a comparative determination of liquid transport capability in the various directions within a material. A preferred embodiment of the present invention has an absorbent core construction in which the expanding material has a non-isotropic liquid transport capability (with a preferential transport direction perpendicular to the plane of the absorbent core) while the other materials used in the absorbent core have essentially isotropic liquid transport capabilities.

The liquid transport capabilities of the individual components of the absorbent core do not have to be inherent material properties but can be influenced. Means commonly used in the art to influence liquid transport capability are for example compression, formation of barriers by heat, bonding or for example impregnation of the materials. For example the absorbent element 44 could be provided with a liquid transport retarding capability along a line encircling the central region of the absorbent element 44 such that the concentration of absorbed liquid in the central region is promoted.

Optionally, a sanitary napkin may have flaps which are adjacent to and extend laterally from the side edge of the absorbent core. The flaps are configured to drape over the edges of the wearer's panties in the crotch region so that the flaps are disposed between the edges of the wearer's panties and the wearer's thighs. The flaps help serve to prevent soiling of the wearer's body and panties to keep the sanitary napkin properly positioned in the panty.

The flaps may be constructed of various materials and according to various designs all well known in the art. Preferred flaps that are suitable or adaptable to the sanitary napkin 20 of the present invention are disclosed in U.S. Pat. No. 4,687,478 issued to Van Tilburg on Aug. 18, 1987 and U.S. Pat. No. 4,589,876 issued to Van Tilburg on May 20, 1986. Alternatively, a sanitary napkin may comprise components that naturally wrap the sides of a wearer's panties.

Characteristics and features usual for diapers or incontinence articles are well known in the art of absorbent articles and will be included in such articles. This includes without limitation elastics, closure mechanisms, leg barriers and others which are known in the art.

Another optional component that can be included in the absorbent articles of the present invention is an odour control means; any suitable odour control means can be incorporated in the article of the present invention in any desired form such as granuals, capsules/microcapsules, powder or impregnation according to techniques well known in the art.

The absorbent article of the present invention is produced and packaged as a conventional essentially flat product and may be therefore manufactured and packaged more easily than a conventional elasticated or pre-formed article. Since the tridimensional structure is formed only during use, the article of the present invention is also easier and more comfortably to wear.

## Claims

1. Use of an absorbent, expanding material for absorbing liquids in an absorbent article, said article comprising a topsheet, a backsheet, and an essentially planar absorbent core disposed between said topsheet and said backsheet, said absorbent core comprising said absorbent, expanding material and said absorbent core having within the plane of said absorbent core a central region and a peripheral region characterized in that said absorbent, expanding material is used to maintain said absorbed liquid concentrated in said central region of said absorbent core.

2. Use according to claim 1 wherein said absorbent, expanding material expands upon initial absorption of liquid by a volume which is larger than the volume of absorbed liquid, thereby creating additional void volume.

3. Use according to any of the preceding claims wherein said absorbent, expanding material extends throughout the central region of said absorbent core.

4. Use according to any of the preceding claims wherein said absorbent, expanding material does not extend into the peripheral region of said absorbent core.

5. Use according to any of the preceding claims wherein said absorbent, expanding material expands upon absorption of liquid primarily in a direction perpendicular to said, essentially planar absorbent material.

6. Use according to claim 5 wherein said absorbent, expanding material expands in a direction from said absorbent core towards said topsheet.

7. Use according to any of the preceding claims wherein said absorbent, expanding material comprises a regenerated cellulose sponge material, preferably consists of a regenerated cellulose sponge material.

8. Use according to claim 7 wherein said cellulose sponge material has incisions in a direction perpendicular to said essentially planar absorbent core and preferably comprises apertures in said cellulose sponge material on the surface of said cellulose sponge material which is closest to said topsheet.

9. Use according to any of the preceding claims wherein each material comprised in said absorbent core has a maximum planar liquid transport capability in a direction within said essentially planar absorbent core and each material in said absorbent core has a maximum perpendicular liquid transport capability in a direction perpendicular to said essentially planar absorbent core and wherein for at least 50 %, preferably at least 95 %, by volume of said material the value of said maximum planar liquid transport capability is smaller than the value of said maximum perpendicular liquid transport capability.

10. Use according to any of the preceding claims wherein said absorbent article is a sanitary napkin.

11. Absorbent article for absorbing liquids, said article comprising a topsheet, a backsheet, and an essentially planar absorbent core disposed between said topsheet and said backsheet, said absorbent core comprising an absorbent, expanding material to maintain said absorbed liquid concentrated in said central region of said absorbent core, said absorbent core having within the plane of said absorbent core a central region and a peripheral region characterized in that each material comprised in said absorbent core has a maximum planar liquid transport capability in a direction within said essentially planar absorbent core and each material in said absorbent core has a maximum perpendicular liquid transport capability in a direction perpendicular to said essentially planar absorbent core, wherein for at least 50 % by volume of said material, which extends into said peripheral region, the value of said maximum planar liquid transport capability is smaller than the value of said maximum perpendicular liquid transport capability.

12. Absorbent article according to claim 11 which is a baby diaper or an adult incontinence article.
